# FASCICULE DE BREVET EUROPEEN

(11) **EP 0 701 450 B1**
(45) Date de publication et mention de la délivrance du brevet: **12.08.1998**
(21) Numéro de dépôt: 94913650.1
(22) Date de dépôt: 15.04.1994
(51) Int. Cl.: A61K 48/00, C12N 15/86

(54) **VIRUS RECOMBINANTS ET LEUR UTILISATION EN THERAPIE GENIQUE**
REKOMBINANTE VIREN UND IHRE VERWENDUNG BEI DER GENTHERAPIE
RECOMBINANT VIRUSES AND THEIR USE IN GENE THERAPY

(30) Priorité: 30.04.1993 FR 9305125
(43) Date de publication de la demande: 20.03.1996
(73) Titulaire: RHONE-POULENC RORER S.A., 92160 Antony (FR)
(72) Inventeur: BENOIT, Patrick, F-75014 Paris (FR); DENEFLE, Patrice, F-94100 Saint-Maur (FR); PERRICAUDET, Michel, F-28320 Ecrosnes (FR); SEGURET, Sandrine, F-78180 Montigny-le-Bretonneux (FR); VIGNE, Emmanuelle, F-94200 Ivry-sur-Seine (FR)
(74) Mandataire: Le Pennec, Magali
(86) Numéro de dépôt international: FR9400422
(87) Numéro de publication internationale: WO9425073

(56) Documents cités:
- WO-A-86/04920
- WO-A-90/06757
- WO-A-92/05253
- WO-A-92/07945
- WO-A-93/01286
- WO-A-93/03769
- EMBASE DATABASE,Amsterdam,Ab.No. 92002545 Frolkis V.V. et al. 'Effect of transfer of...'
- DATABASE WPI Week 8949, Derwent Publications Ltd., London, GB; AN 89-364119 [49] & US,A,7 365 567 (US DEPT HEALTH & HUMAN) 19 Septembre 1989
- DATABASE WPI Week 8845, Derwent Publications Ltd., London, GB; AN 88-320037 [45] & JP,A,63 237 795 (OTSUKA PHARM KK) Octobre 1988
- DATABASE WPI Week 9149, Derwent Publications Ltd., London, GB; AN 91-361682 [49] & US,A,7 601 931 (US DEPT HEALTH & HUMAN ET AL.) Novembre 1991
- PROCEEDINGS OF THE NATIONAL ACADEMY OF SCIENCES OF USA., vol.86, no.22, 1989, WASHINGTON US pages 8803 - 8807 E.S.FENJVES ET AL. 'Systemic distribution of apolipoprotein E ...'
- DATABASE WPI Week 8631, Derwent Publications Ltd., London, GB; AN 86-202279 [31] & JP,A,61 135 587 (NORIINSHO KK) Juin 1986
- DATABASE WPI Week 8624, Derwent Publications Ltd., London, GB; AN 86-150217 [24] & AU,D,4 751 385 (MITSUBICH CHEM IND KK ET AL.) Avril 1986
- BIOTECHNOLOGY ABSTRACTS,London,GB,Ab.No. 93-11561 Williams R S 'Southwestern Internal Medical Confer. ..'

## Description

La présente invention concerne de nouveaux virus recombinants, leur préparation et leur utilisation en thérapie génique, pour le transfert et l'expression in vivo de gènes désirés. Plus précisément, elle concerne de nouveaux virus recombinants comprenant un gène inséré dont l'expression in vivo permet de réguler les niveaux plasmatiques d'apolipoprotéines. La présente invention concerne également des compositions pharmaceutiques comprenant lesdits virus recombinants. Plus particulièrement, la présente invention concerne des virus recombinants défectifs et leur utilisation pour la prévention ou le traitement des pathologies liées aux dyslipoprotéinémies, qui sont conues pour leur conséquences graves au niveau cardiovasculaire et neurologique.

Les dyslipoprotéinémies sont des désordres du métabolisme des lipoprotéines responsables du transport dans le sang et les fluides périphériques de lipides comme le cholestérol et les triglycérides. Elles conduisent à des pathologies importantes, liées respectivement à l'hypercholestérolémie ou l'hypertriglycéridémie, telles que notamment l'athérosclérose. L'athérosclérose est une maladie complexe, polygénique, qui est définie sur le plan histologique par des dépôts (plaques lipidiques ou fibro-lipidiques) de lipides et d'autres dérivés sanguins dans la paroi des grosses artères (aorte, artères coronaires, carotide). Ces plaques, plus ou moins calcifiées selon l'avancement du processus, peuvent être jumelées à des lésions et sont liées à l'accumulation dans les artères de dépots graisseux constitués essentiellement d'esters de cholestérol. Ces plaques s'accompagnent d'un épaississement de la paroi artérielle, avec hypertrophie du muscle lisse, apparition de cellules spumeuses et accumulation de tissu fibreux. La plaque athéromateuse est très nettement en relief sur la paroi, ce qui lui confère un caractère sténosant responsable des occlusions vasculaires par athérome, thrombose ou embolie qui surviennent chez les patients les plus atteints. Les hypercholestérolémies peuvent donc conduire à des pathologies cardio-vasculaires très graves telles que l'infarctus, la mort subite, la décompensation cardiaque, les accidents cérébro-vasculaires, etc.

Il est donc particulièrement important de pouvoir disposer de traitements permettant de diminuer dans certaines situations pathologiques les taux de cholestérol plasmatique voire de stimuler l'efflux de cholestérol (transport inverse du cholestérol) au niveau des tissus périphériques afin de décharger les cellules ayant accumulé du cholestérol dans le contexte de la formation d'une plaque d'athérome. Le cholestérol est véhiculé dans le sang par diverses lipoprotéines dont les lipoprotéines de faible densité (LDL) et les lipoprotéines de haute densité (HDL). Les LDL sont synthétisées au niveau du foie et permettent d'approvisionner les tissus périphériques en cholestérol. Au contraire, les HDL captent le cholestérol au niveau des tissus périphériques et le transportent vers le foie où il est stocké et/ou dégradé.

Actuellement, les dyslipidémies et en particulier les hypercholestérolémies sont traitées essentiellement au moyen de composés agissant soit sur la biosynthèse du cholestérol (inhibiteurs de l'hydroxyméthylglutaryl-coenzymeA reductase, les statines), soit sur la captation et l'élimination du cholestérol biliaire (les séquestrants ou résines), soit encore sur la lipolyse par un mode d'action qui reste à élucider sur le plan moléculaire (les fibrates). Par conséquent, toutes les grandes classes de médicaments, qui ont été utilisées dans cette indication (séquestrants, fibrates ou statines), s'adressent seulement à l'aspect préventif de la formation de la plaque d'athérome et non en fait au traitement de l'athérome. Les traitements actuels de l'athérome, post accident coronarien, ne sont que palliatifs puisqu'ils n'interviennent pas sur l'homéostase du cholestérol et qu'ils sont des actes chirurgicaux (by-pass coronarien, angioplastie).

La présente invention constitue une nouvelle approche thérapeutique pour le traitement des pathologies liées aux dyslipoprotéinémies. Elle propose une solution avantageuse aux inconvénients de l'art antérieur, en démontrant la possibilité de traiter les pathologies liées aux dyslipoprotéinémies par la thérapie génique, par le transfert et l'expression in vivo de gènes capables de réguler les niveaux plasmatiques d'apolipoprotéines. L'invention offre ainsi un moyen simple permettant un traitement spécifique et efficace de ces pathologies.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

La présente invention constitue une nouvelle approche thérapeutique pour le traitement des pathologies liées aux dyslipoprotéinémies consistant à transférer et à exprimer in vivo des gènes capables de réguler les niveaux plasmatiques d'apolipoprotéines. La présente invention résulte également de la mise en évidence que les adénovirus constituent des vecteurs particulièrement efficaces pour le transfert et l'expression de tels gènes. En particulier, la présente invention montre que l'utilisation d'adénovirus recombinants comme vecteurs permet d'obtenir des niveaux d'expression suffisamment élevés de ces gènes pour produire l'effet thérapeutiqe recherché. La présente invention offre ainsi une nouvelle approche pour le traitement et la prévention des pathologies cardiovasculaires et neurologiques liées aux dyslipoprotéinémies.

Un premier objet de l'invention réside donc dans un adénovirus recombinant défectif contenant au moins un gène inséré dont l'expression permet de réguler les niveaux d'apolipoprotéine in vivo.

L'invention a également pour objet l'utilisation d'un tel adénovirus recombinant défectif pour la préparation d'une composition pharmaceutique destinée au traitement ou à la prévention des pathologies liées aux dyslipoprotéinémies.

La présente invention repose plus particulièrement sur la mise en évidence que les virus de type adénovirus sont capables de transférer et d'exprimer des gènes codant pour des apolipoprotéines au niveau du foie, et de sécréter lesdites apolipoprotéines dans le système circulatoire où elles exercent leur activité. Les exemples présentés plus loin indiquent que les adénovirus sont capables, selon le mode d'administration, de transférer et d'exprimer efficacement, pour une durée importante et sans effet cytopathologique, les gènes codant pour l'apolipoprotéine AI ou l'apolipoprotéine AIV.

Au sens de la présente invention, le terme "adénovirus défectif" désigne un adénovirus incapable de se répliquer de façon autonome dans la cellule cible. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par le gène inséré. Préférentiellement, le virus défectif conserve néanmoins les séquences de son génome qui sont nécessaires à l'encapsidation des particules virales.

Il existe différents sérotypes d'adénovirus, dont la structure et les propriétés varient quelque peu. Néanmoins, ces virus ne sont pas pathogènes pour l'homme, et notamment les sujets non immuno-déprimés. Parmi ces sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Au sens de la présente invention, le gène inséré capable de réguler les niveaux d'apolipoprotéine in vivo peut être un gêne codant pour une apolipoprotéine ou pour un produit protéique ayant une activité de type apolipoprotéine, ou également un gène antisens, dont l'expression dans la cellule cible permet de contrôler l'expression de gènes ou la transcription d'ARNm cellulaires codant pour des apolipoprotéines. Le terme produit protéique ayant une activité de type apolipoprotéine désigne tout mutant, fragment ou peptide possédant au moins une propriété biologique d'une apolipoprotéine, ainsi que tous variants naturels des apolipoprotéines.

Le gène inséré peut être un fragment d'ADNc, d'ADN génomique, ou une construction hybride consistant par, exemple en un ADNc dans lequel seraient insérés un ou plusieurs introns. Il peut également s'agir de séquences synthétiques ou semisynthétiques.

Parmi les gènes insérés au sens de la présente invention, on peut citer plus particulièrement les gènes codant pour tout ou une partie active des apolipoprotéines AI, AIV ou E.

L'apolipoprotéine AI est une protéine constituée de 243 acides aminés, synthétisée sous la forme d'une prépropeptide de 267 résidus, ayant une masse moléculaire de 28.000 daltons. Elle est synthétisée chez l'homme spécifiquement dans le foie et l'intestin et elle constitue la protéine essentielle des particules HDL (70% de leur masse en protéines). Elle est abondante dans le plasma (1.0-1.2 g/l). Son activité la mieux caractérisée sur le plan biochimique est l'activation de la lécithine-cholestérol acyl-transférase (LCAT), mais de nombreuses autres activités lui sont attribuées, comme notamment la stimulation de l'efflux de cholestérol cellulaire. Le rôle physiologique de l'apolipoprotéine AI semble contrebalancé par l'apolipoprotéine AII puisque chez l'homme, le rapport des deux concentrations plasmatiques (AII/AI) est très étroitement corrélé avec le risque coronarien. L'apolipoprotéine AI joue un rôle majeur dans la résistance à l'athérosclérose, probablement lié au transport inverse du cholestérol, puisque la seule expression de cette apolipoprotéine dans des souris transgéniques permet de réduire de 40 fois la surface des dépôts lipidiques au niveau de l'aorte par rapport à des souris contrôles (Rubin et al. 1993 Science, In Press). Son gène, long de 1863 bp, a été cloné et séquencé (Sharpe et al., Nucleic Acids Res. 12(9) (1984) 3917). Parmi les produits protéiques à activité de type apolipoprotéine AI, on peut citer notamment les variants naturels décrits dans l'art antérieur (tableau ci-après).

| Variant: | Mutation | Variant | Mutation |
|---|---|---|---|
| Milano | Arg173Cys | Norway | Glu136Lys |
| Marburg | Lys107Ø | | Pro165Arg |
| Munster2B | Ala158Glu | | Pro3His |
| Giessen | Pro143Arg | | Arg10Leu |
| Munster3A | Asp103Asn | | Gly26Arg |
| Munster3B | Pro4Arg | | Asp89Glu |
| Munster3C | Pro3Arg | | Lys107Met |
| Munster3D | Asp213Gly | | Glu139Gly |
| Munster4 | Glu198Lys | | Glu147Val |
| Yame | Asp13Tyr | | Ala158Glu |
| | Asp213Gly | | Glu169Gln |
| | | | Arg177His |

L'apolipoprotéine AIV (apoAIV) est une protéine constituée de 376 acides aminés, synthétisée spécifiquement dans l'intestin sous la forme d'un précurseur de 396 résidu. La protéine plasmatique, est relativement abondante (0.16 g/l) et a une masse moléculaire de 46.000 daltons. Elle est un composant majeur des chylomicrons sécrétés dans la lymphe, mais elle présente la particularité d'être majoritairement sous forme non associée avec des lipoprotéines dans le plasma (R.B. Weinberg et Coll., 1983, J. Lipid. Research, 24 : 52-59). Par ailleurs, l'apoAIV plasmatique est polymorphe, bien que la nature de ce polymorphisme soit encore inconnue (G. Utermann et Coll., 1982, J. Biol. Chem. 257 : 501-507). Le rôle physiologique de l'apoAIV demeure également assez peu connu. On sait qu'elle peut activer in vitro la lécithin-cholestérol-acyltransférase (LCAT) (Steinmetz et Coll., 1985, J. Biol. Chem., 260 : 2258-2264) et qu'elle peut, comme l'apolipoprotéine AI, interférer avec la fixation des particules de HDL sur les cellules endothéliales aortiques bovines (Savion et Coll., 1987, Eur. J. Biochem., 257 : 4171-4178). Ces deux activités indiquent que l'apoAIV intervient très vraisemblablement comme médiateur du transport inverse du cholestérol. Le gène de l'apoAIV a été cloné et décrit dans l'art antérieur (voir notamment WO 92/05253). Parmi les produits protéiques à activité de type apolipoprotéine AIV, on peut citer notamment les fragments et dérivés décrits dans la demande de brevet FR 92 00806.

L'apolipoprotéine E comprend 317 résidus dont 18 correspondent au peptide signal. Il n'y a pas de propeptide. Le gène de l'apoE a été cloné et séquencé (environ 3600bp) et code pour un ARNm de 1163bp. L'apoE est répartie dans le plasma entre les particules VLDL et HDL. Elle représente environ 10-20% des proteines des VLDL et 2% des protéines des HDL. Les HDL-E constituent une sous-classe distincte de HDL. La concentration plasmatique de l'apoE est d'environ 0.05 g/l. L'apoE est synthétisée sous la forme d'une sialo-protéine qui est ensuite désialilée dans le plasma. La synthèse de l'apoE est réalisée par le foie et faiblement par l'intestin. Cependant, contrairement aux autres apolipoprotéines, l'apoE est également synthétisée dans de nombreux autres tissus (cerveau, rein, surrénales, cellules réticulo-endothéliales...). L'apoE reconnait avec une très forte affinité le récepteur aux LDL (apoB/E receptor) mais également un autre récepteur sur les cellules héptiques ne reconnaissant pas l'apoB (chylomicron/remnant receptor).

Un polymorphisme a été mis en évidence sur la base de mobilités electrophorètiques différentes. Six phénotypes majeurs (E2/2, E2/3, E2/4, E3/4, E3/3, E4/4) ont ainsi été décrits. Selon les études menées sur de grandes populations caucasiennes, la prévalence des allèles correspondants serait de 14-15% pour ε4, de 74-78% pour ε3 et 8-12% pour ε2. Un écart est trouvé chez les Finlandais pour lesquels ε4 est plus abondant (23%) et ε2 moins abondant(4%). L'allèle normal est ε3. L'allèle ε2 correspondrait à la dyslipoprotéinémie de type III (phénotype E2/2), maladie associée à une augmentation du cholestérol et des triglyrérides, des xanthomes et à une athérosclerose précoce. Une association entre l'allèle ε4 et la maladie d'Alzheimer familiale a été rapportée récemment (Strittmatter et al. P.N.A.S. 99 (1993) 1977). Plus récemment la destruction du gène de l'apoE dans la souris a montré l'apparition d'une hypersusceptibilité à l'athérosclérose (E. Rubin et al. Cell 1992).

Au sens de l'invention, le terme "gène inséré" désigne également des séquences antisens, dont l'expression dans la cellule cible permet de contrôler l'expression de gènes ou la transcription d'ARNm cellulaires codant pour des apolipoprotéines. De telles séquences peuvent par exemple être transcrites, dans la cellule cible, en ARN complémentaires d'ARNm cellulaires et bloquer ainsi leur traduction en protéine.

Parmi les séquences antisens utilisables dans le cadre de l'invention, on peut citer plus particulièrement toute séquence antisens permettant de diminuer les niveaux de production d'apolipoprotéine AII, ainsi qu'illustré dans les exemples.

Généralement, le gène inséré comprend également des séquences permettant son expression dans la cellule infectée. Il peut s'agir des séquences qui sont naturellement responsables de l'expression dudit gène lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences de gènes eucaryotes ou viraux. A titre d'exemple, il peut s'agir de séquences promotrices issues du génome de la cellule que l'on désire infecter, ou du génome d'un virus, et notamment, les promoteurs des gènes E1A, MLP d'adénovirus, le promoteur CMV, LTR-RSV, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Par ailleurs, lorsque le gène inséré code pour une apolipoprotéine ou pour un produit protéique à activité de type apolipoprotéine, celui-ci comprend généralement, en amont de la séquence codante, une séquence signal dirigeant le polypeptide synthétisé dans les voies de sécrétion de la cellule cible. Cette séquence signal peut être la séquence signal naturelle de l'apolipoprotéine, mais il peut également s'agir de toute autre séquence signal fonctionnelle, ou d'une séquence signal artificielle.

Les adénovirus recombinants défectifs selon l'invention peuvent être préparés par recombinaison homologue entre un adénovirus et un plasmide portant entre autre le gène que l'on désire insérer. La recombinaison homologue se produit après cotransfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %).

Ensuite, les vecteurs qui se sont multipliés sont récupérés et purifiés selon les techniques classiques de biologie moléculaire.

La présente invention concerne également une composition pharmaceutique comprenant un ou plusieurs adénovirus recombinants défectifs tels que décrits précédemment. De telles compositions peuvent être formulées en vue d'une administration par voie topique, orale, parentérale, intranasale, intraveineuse, intramusculaire, sous-cutanée, intraoculaire, etc.

Préférentiellement, la composition contient des véhicules pharmaceutiquement acceptables pour une formulation injectable.

Dans leur utilisation pour le traitement des pathologies liées aux dyslipoprotéinémies, les adénovirus recombinants défectifs selon l'invention peuvent être administrés selon différents modes, et notamment par injection intraveineuse, . Préférentiellement, ils sont injectés au niveau de la veine porte.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une suspension de virions, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

La présente invention offre ainsi un moyen très efficace pour le traitement ou la prévention des pathologies liées aux dyslipoprotéinémies en particulier dans le domaine des affections cardiovasculaires comme l'infarctus du myocarde, l'angor, la mort subite, la décompensation cardiaque, les accidents cérébro-vasculaires, ou dans le domaine des affections neurologiques où certaines apolipoprotéines comme l'apoE semblent jouer un rôle important (maladies du viellissement neuronal, Alzheimer familial, régénération neuronale). Plus généralement, cette approche offre un moyen d'intervention thérapeutique très prometteur pour chaque cas où un déficit d'ordre génétique ou métabolique d'une apolipoprotéine plasmatique peut être corrigé.

En outre, ce traitement peut concerner aussi bien l'homme que tout animal tel que les ovins, les bovins, les animaux domestiques (chiens, chats, etc), les chevaux, les poissons, etc.

La présente invention est plus complètement décrite à l'aide des exemples qui suivent, qui doivent être considérés comme illustratifs et non limitatifs.

### LEGENDE DES FIGURES

Figure 1 : Stratégie de construction du plasmide pXL2099.
Figure 2 : Stratégie d'amplification du cDNA de l'apoAI.
Figure 3 : Construction du plasmide pXL2235.
Figure 4 : Représentation des plasmides pXL2244 et pXL2263.
Figure 5 : Stratégie de construction du minigène d'ApoAI.
Figure 6 : Représentation du plasmide pXL2336.
Figure 7 : Représentation du plasmide pXL2375.
Figure 8 : Effet de l'injection d'un adénovirus ApoAI sur le profil des HDL.
Figure 9 : Effet de l'injection d'un adénovirus ApoAI sur les niveaux plasmatiques d'ApoAI.
Figure 10 : Représentation du plasmide pXL2335.

### TECHNIQUES GENERALES DE BIOLOGIE MOLECULAIRE

Les méthodes classiquement utilisées en biologie moléculaire telles que les extractions préparatives d'ADN plasmidique la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d 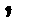 ADN peuvent être séparés selon leur taille par électrophorèse en gels d 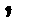 agarose ou d'acrylamide, extraits au phénol ou par un mélange phénol/chloroforme, précipités à l 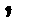 éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d 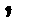 E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### EXEMPLES

### Exemple A : Construction d'un adénovirus recombinant défectif contenant le gène de l'apolipoprotéine AI:

Comme indiqué avant, les adénovirus recombinants défectifs ont été préparés par recombinaison homologue entre un adénovirus et un plasmide portant entre autre le gène que l'on désire insérer, après co-transfection dans une lignée cellulaire appropriée.

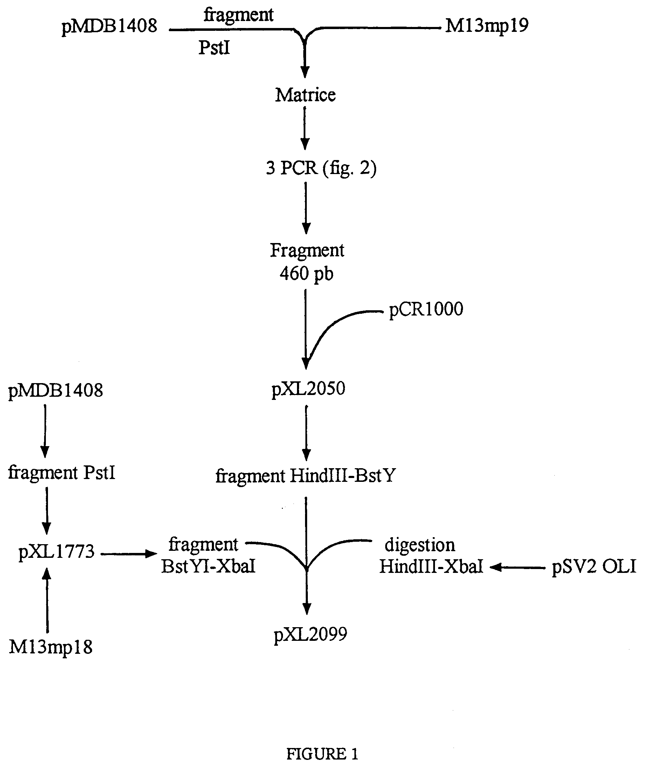

### A1 Préparation des plasmides portant le gène de l'apoAI (voir figures 1-4)

Les plasmides utilisés pour générer, par recombinaison homologue, les adénovirus recombinants exprimant le gène de l'apolipoprotéine AI humaine ont été construits comme suit :

### 1. Construction du plasmide pXL2236

Le plasmide pXL2236 contient notamment l'ADNc codant pour la préproApoAI sous le contrôle du promoteur CMV, les signaux de polyadénylation du virus SV40, ainsi qu'une région d'adénovirus permettant la recombinaison homologue.

Il a été construit de la manière suivante :

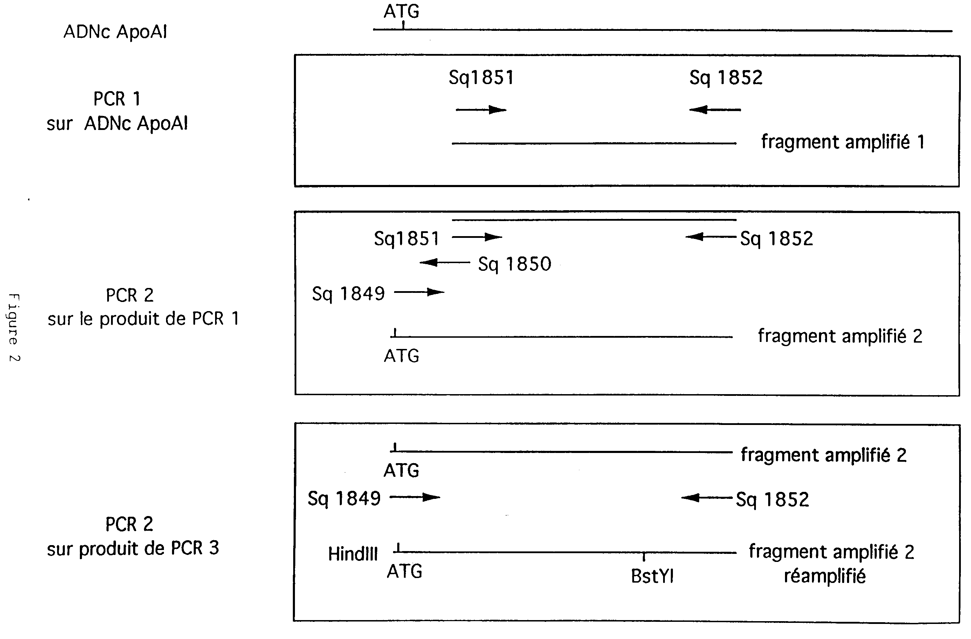

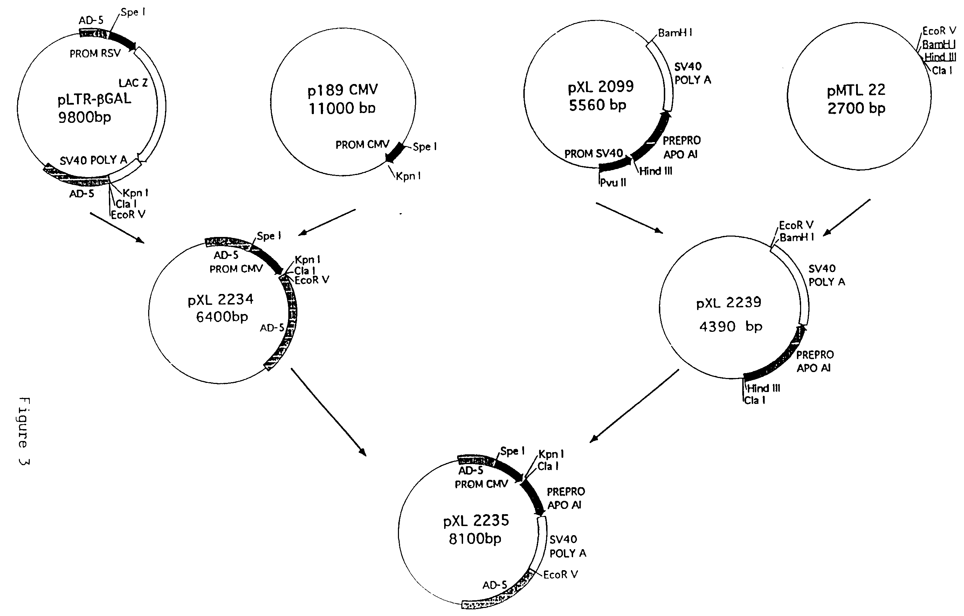

Le fragment d'ADN correspondant au cDNA de l'apoAI humaine a été isolé sous forme d'un fragment PstI à partir du vecteur pMD1408 décrit par S.Law et B.Brewer Jr. (PNAS 81 (1984) 66-70). Ce fragment a été introduit dans le plasmide M13mp19 (figure 1). Ce fragment a ensuite été modifié par PCR successives (figure 2) pour introduire un site HindIII en position 5'. Ainsi, une première réaction de PCR a été réalisée sur le plasmide obtenu ci-dessus au moyen des oligonucléotides Sq1851 (SEQ ID n° 1) et Sq1852 (SEQ ID n° 2). L'ADN issu de cette PCR a été réamplifié dans une seconde PCR avec les oligonucléotides Sq1849 (SEQ ID n° 3), Sq1850 (SEQ ID n° 4), Sq1851 et Sq1852. Une troisième PCR a enfin été pratiquée à partir du résultat de la seconde PCR avec les oligonucléotides Sq1849 et Sq1852. Le fragment d'environ 460pb obtenu a été cloné dans le plasmide PCR1000 (Invitrogen) et sa séquence vérifiée. Le plasmide résultant a été appelé plasmide pXL2050 (figure 1). Les fragments HindIII-BstY1 de pXL2050 et BstY1-XbaI de pXL1773 (fragment PstI du plasmide pMDB1408 inséré dans M13mp18) ont ensuite été clonés dans le plasmide pSV2 OLI (Subramani et al., MCB 1 (1991) 854) préalablement digéré par HindIII-XbaI, pour générer le plasmide pXL2099 (figure 3).

Le fragment SpeI-KpnI portant le promoteur du cytomégalovirus (CMV) a ensuite été isolé à partir du vecteur pEBVDdyadCMVlacIn (p189 CMV, Biard et al., Biochemichal et Biophysical Acta 1130 (1992) 68), puis inséré aux sites correspondants du vecteur pRSV-βgal (Stratford-Perricaudet et al., J. Clin. Invest. 90 (1992) 626). Le plasmide obtenu a été désigné pXL2234. Lors de cette étape, les régions codantes RSV LTR et lac Z sont éliminées.
Ensuite, le fragment Hind III-BamHI aux extrémités franches contenant la région codant pour la pre-pro ApoI ainsi que les signaux de maturation de l'ARNm du virus SV40 a été isolé à partir du plasmide pXL2099, puis inséré au site EcoRV du plasmide pXL2234.

Le plasmide ainsi obtenu a été désigné pXL2236.

### 2. Construction du plasmide pXL2235

Un second plasmide, désigné pXL2235, a été construit. Ce plasmide contient notamment l'ADNc codant pour la préproApoAI sous le contrôle du promoteur CMV, les signaux de polyadénylation du virus SV40, ainsi qu'une région d'adénovirus permettant la recombinaison homologue.

Il a été obtenu en deux étapes :
- insertion du fragment HindIII-BamHI du plasmide pXL2099 identifié ci-dessus, dans le vecteur pMTL22 (Chambers et al., Gene 68 (1988) 139) digéré par les mêmes enzymes, puis,
- digestion du plasmide obtenu ci-dessus par les enzymes ClaI-EcoRV et insertion du fragment ClaI-EcoRV ainsi isolé dans le plasmide pXL2234 digéré par EcoRV.

Le plasmide ainsi obtenu a été désigné pXL2235 (figure 3).

### 3. Construction du plasmide pXL 2244

Le plasmide pXL2244 contient l'ADNc de l'ApoAI sous le contrôle du promoteur LTR du virus RSV. Il a été construit par insertion du fragment ClaI-EcoRV de pXL2239 dans le vecteur pLTR RSV-βgal digéré par les même enzymes.

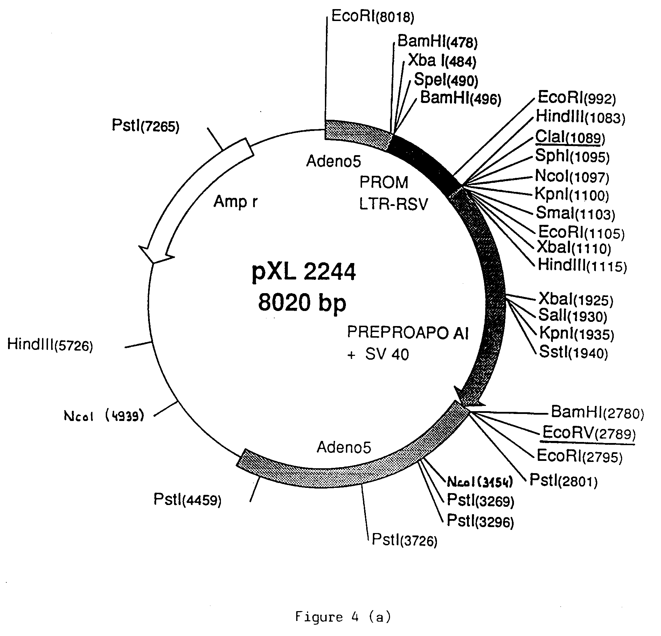

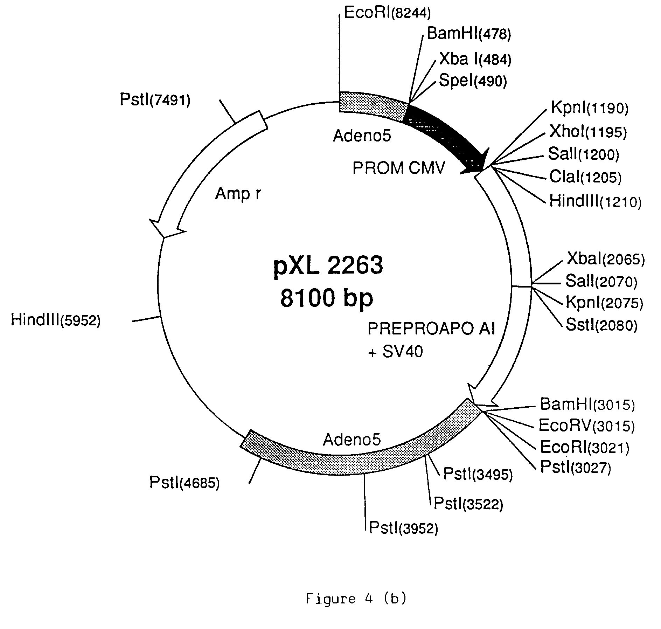

La structure du plasmide pXL2244 est donnée figure 4.

### 4. Construction du plasmide pXL 2263

Le plasmide pXL 2263 est dérivé du vecteur pXL 2235 dans lequel une partie du polylinker séparant le promoteur CMV de l'ADNc de l'Apo AI (fragment KpnI) a été délété. Il a été construit en deux étapes:

Tout d'abord, le fragment Hind III - BamHI de pXL 2099 contenant l'ADNc de l'Apo AI et le fragment de SV40 a été cloné aux même sites d'un plasmide pBluescript, pour donner naissance au plasmide pXL2245.

Ensuite, le fragment KpnI contenant l'ADNc de l'Apo AI de ce plasmide a été recloné dans le bon sens dans le vecteur pXL2235 digéré par KpnI.

La structure du plasmide pXL2263 est donnée figure 4.

### 5. Construction du plasmide pXL2336

Le plasmide pXL2336 contient une construction hybride entre une séquence génomique contenant le promoteur, le premier exon et le premier intron du gène de l'apoAI et la séquence de l'ADNc de l'apoAI.

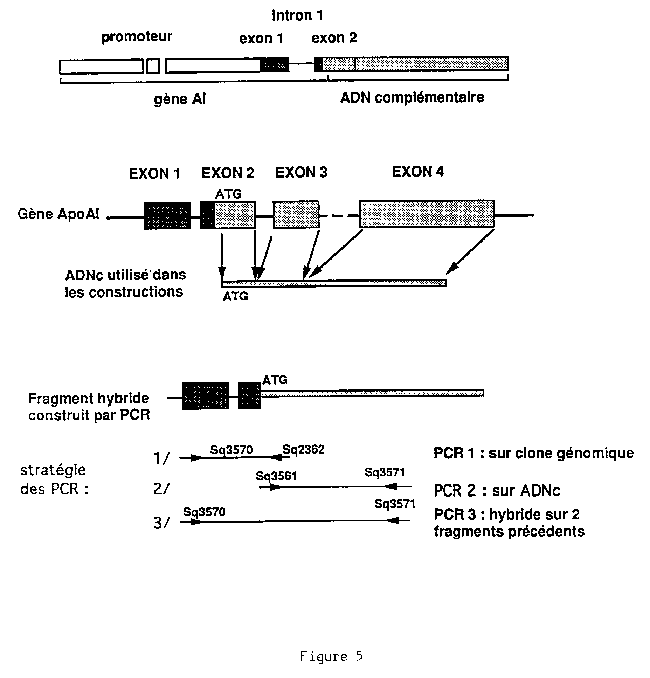

Pour cela la séquence hybride décrite en figure 5 a été construite par PCR comme décrit ci-après.

Les amorces utilisés sont Sq3561 (SEQ ID n° 5) et Sq3571 (SEQ ID n° 6) qui amplifent la séquence de la base 14 à la base 441 de l'ADNc de l'ApoAI ainsi que Sq3570 (SEQ ID n° 7) et Sq3562 (complémentaire de Sq3561) qui amplifient un fragment s'étendant du promoteur, en incluant un site AflII unique, au début du second exon. Les deux fragments, qui sont donc complémentaires par les amorces Sq3561 et Sq3562, ont ensuite été purifiés sur gel mélangé et utilisés comme matrice pour une seconde PCR avec les amorces Sq3571 et Sq3570. Le fragment hybride est ensuite cloné dans pCR II (Invitrogen), résultant dans le plasmide pXL2314, et sa séquence vérifiée.

Un fragment de pXL2314 coupé par XhoI, rempli à la Klenow puis recoupé par AflII a ensuite été cloné dans le plasmide pXL2068, préalablement digéré par HindIII, rempli à la Klenow et recoupé par AflII. Le plasmide pXL2068 contient le promoteur de l'ApoAI, du site KpnI situé vers la base -2200 jusqu'au site KpnI situé après le site d'initiation de la transcription (base +93), cloné dans un plasmide pUC19 avec l'extrémité 5' du promoteur du coté du site EcoRI, puis recloné par les sites XbaI et SstI dans le plasmide pSL301 (Invitrogen). Le plasmide résultant a été appelé pXL2318.

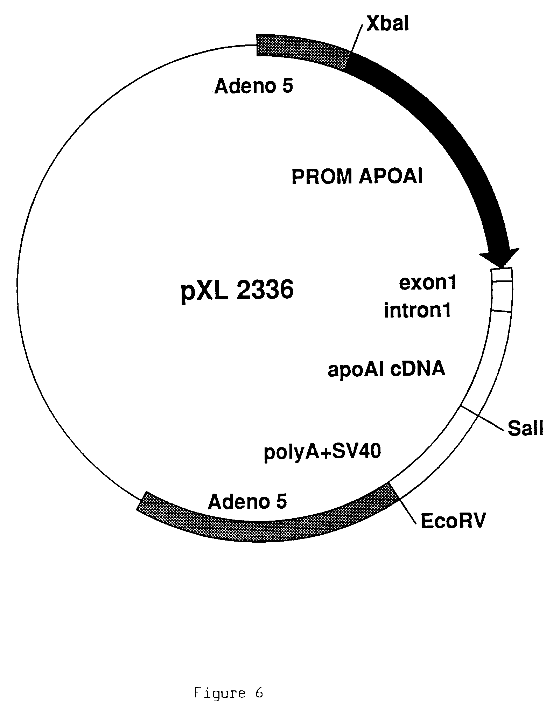

Un fragment XbaI-Bsu36I de pXL2318 est enfin cloné dans le plasmide pXL2263 décrit précedemment et coupé par SpeI puis Bsu36I de façon partielle. Le plasmide résultant est finalement appelé pXL2336 et sa structure donnée en figure 6.

### 6. Construction du plasmide pXL2375

Ce plasmide contient l'ADNc de l'ApoAI sous le contrôle du promoteur CMV s'étendent jusqu'au site 5' donneur d'épissage lié aux 107 paires de bases les plus 3' de l' intron synthétique décrit par O'Gorman et al (Science, 1991, 251:1351-1355) qui fournissent alors le site 3' d'épissage.

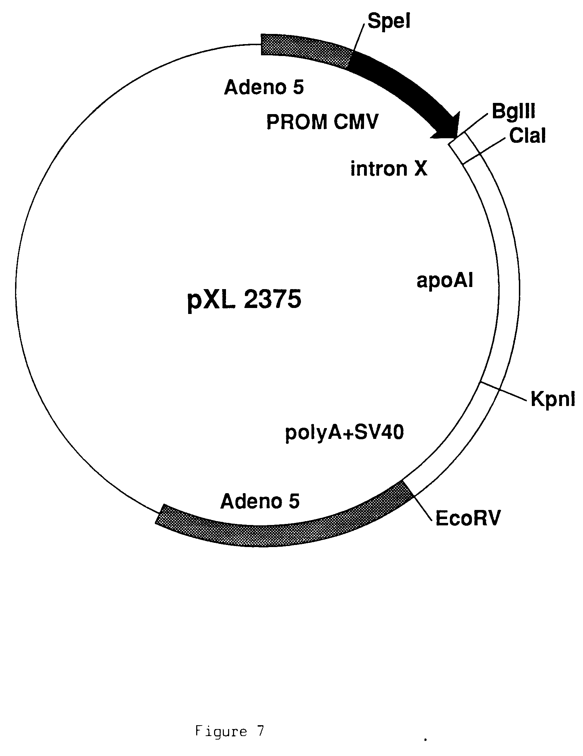

Ce promoteur est sorti du plasmide pAICMVIXCAT.3 (Philip et al., Molecular and Cellular Biology, sous presse) par les sites XbaI et ClaI et cloné aux mêmes sites dans le vecteur pXL2263 pour donner le plasmide pXL2375. Sa carte est donnée en figure 7.

### A2. Expression d'apoAI in vitro

Les vecteurs décrits en A1 ont été testés pour leur fonctionalité, par expression dans les lignées Cos1 ou Cos7. Pour cela, les vecteurs ont été introduits dans les cellules par électroporation, selon le protocole décrit par P.Benoit et al, Journal of Immunology 150 (1993) 707.

48 heures après la transfection, les surnageants cellulaires ont été récupérés et testés pour leur contenu en Apo AI par un test ELISA. Pour cela, des plaques Immulon II (Dynatech) ont été revêtues avec un anticorps monoclonal anti ApoAI (10mg/ml dans du tampon carbonate pH 9,6), par incubation une nuit à 4°C, puis saturées par 2% BSA en PBS pH 7,4 une heure à 37°C. Les surnageants cellulaires ont ensuite été incubes une heure à 37°C, éventuellement après dilution en PBS 2% BSA. La révélation a ensuite été effectuée par incubation une heure à 37°C avec un mélange d'anticorps monoclonaux anti ApoAI marqués à la péroxydase, et dilué au 1/5000. La fixation des anticorps péroxydés est finalement révélée par incubation avec 250µl de TMB (KPL) et lecture des plaques à 630nm.

Les résultats obtenus montre que, dans tous les cas testés, l'expression d'ApoAI peut être détectée.

### A3. Préparation des adénovirus recombinants

Les plasmides préparés en A1 ont ensuite été linéarisés et cotransfectés pour la recombinaison avec le vecteur adénoviral déficient, dans les cellules helper (lignée 293) apportant en *trans* les fonctions codées par les régions E1 (E1A et E11B) d'adénovirus.

L'adénovirus Ad.CMVApoAI a été obtenu par recombinaison homologue in vivo entre l'adénovirus Ad.RSVβgal (Stratford-Perricaudet et al., J. Clin. Invest 90 (1992) 626) et le plasmide pXL2235 selon le protocole suivant : après , Le plasmide pXL2235 linéarisé par l'enzyme EagI et l'adénovirus Ad.RSVβgal linéarisé par ClaI sont co-transfectés dans la lignée 293 en présence de phosphate de calcium pour permettre la recombinaison homologue. Les adénovirus recombinants ainsi générés sont sélectionnés par purification sur plaque. Après isolement, l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.

Les particules virales sont généralement purifiées par centrifugation sur gradient de chlorure de césium selon les techniques connues (voir notamment Graham et al., Virology 52 (1973) 456). L'adénovirus Ad.CMVApoAI est conservé à -80°C dans 20% de glycérol.

Le même protocole a été reproduit avec le plasmide pXL2244 conduisant à l'adénovirus recombinant Ad.RSVApoAI.

### Exemple B : Transfert et expression in vivo du gène de l'apoAI

Le stock de virus que l'on produit ainsi est ensuite utilisé pour l'infection in vivo.

Dans un premier temps, les virus sont utilisés pour l'infection in vivo des hépatocytes chez la souris. Pour cela, les particules virales sont injectées par exemple par la voie portale chez des souris C57B16. Le mode d'injection et la spécificité d'expression ont été testés par une étude préalable réalisée avec des virus exprimant la β-galactosidase (une technique d'injection utilisable a aussi été décrite par Jaffe et al., Nature Genetics, vol.1 (1992) 372). Le niveau d'expression plasmatique d'apoAI humaine est testé par ELISA à l'aide d'anticorps monoclonaux spécifiques de la protéine humaine, et la spécificité de l'expression du gène recombinant est vérifiée à l'aide de sondes oligonucléotidiques humaines spécifiques dans différents tissus (northern blotting et RT-PCR).

Ces virus, nommés respectivement Ad (RSV-ApoAI) et Ad (CMV-ApoAI), sont injectés dans des souris C57B1/6 par voie intra veineuse en utilisant la veine de la queue ou le sinus rétro-orbital. Il sont injectés dans des souris knock-out ApoAI (Williamson et al, 1992, Proc.Natl.Acad.Sci.USA, 89:7134-7138), dans lesquelles l'ApoAI de souris n'est pas exprimée et dans lesquelles le HDL-cholestérol est très faible ce qui permet d'avoir un background très propre pour voir les effets de l'expression de l'ApoAI humaine. Cette expression est testée par un ELISA à l'aide d'anticorps monoclonaux spécifique de la protéine humaine modifié d'après Betard et al (J. Clin. Chem. Clin. Biochem.,1987, 25:893-899).

### B1. Expression dans les souris ApoAI Knock-out

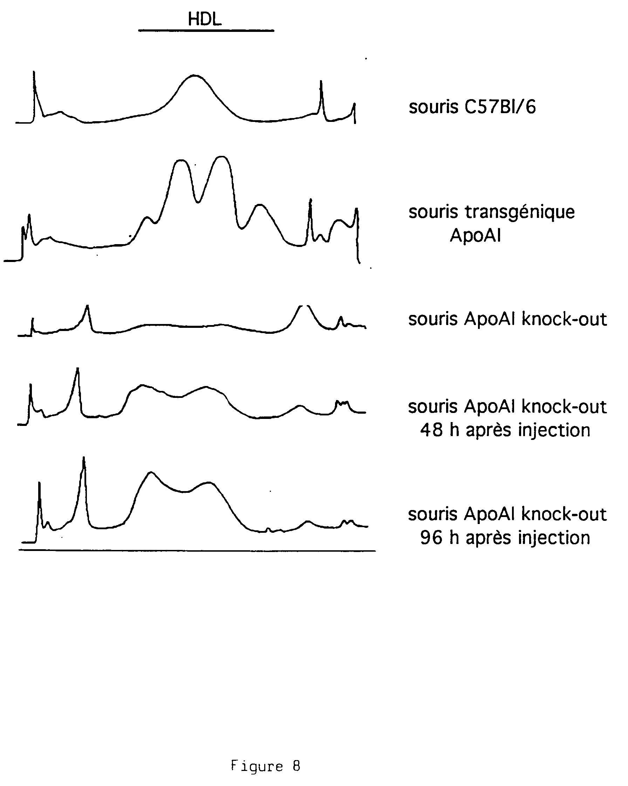

Le virus Ad(CMV-ApoAI) est d'abord injecté dans des souris Knock-out ApoAI qui sont donc déficientes dans l'expression d'ApoAI de souris. Après injection de 10⁹ ufp par la veine de la queue, de l'apoAI humaine est trouvé à la concentration de 0,3mg/dl dans le plasma après 48h et 96h. La concentration en HDL-cholestérol est également mesuré au même temps, ce qui permet de mesurer une augmentation de 8mg/dl avant l'injection à des niveaux de 30-35mg/dl à 48 et 96 heures. Ces souris injectées par Ad(CMV-ApoAI) permettent de mettre en évidence un profil lipoprotéinique correspondant à des HDL de type humain avec une population de HDL polydispersée que l'on retrouve également chez des souris transgéniques pour le gène de l'ApoAI humaine chez qui le composant majeur des HDL est devenu l'ApoAI humaine. Par comparaison chez une souris normale, le profil des HDL correspond à une seule population de particule en taille et chez les souris Knock-out ApoAI le profil est plat dans la région correspondante et il existe une population de HDL de taille anormale (figure 8).

### B2. Expression dans les souris C57B1/6

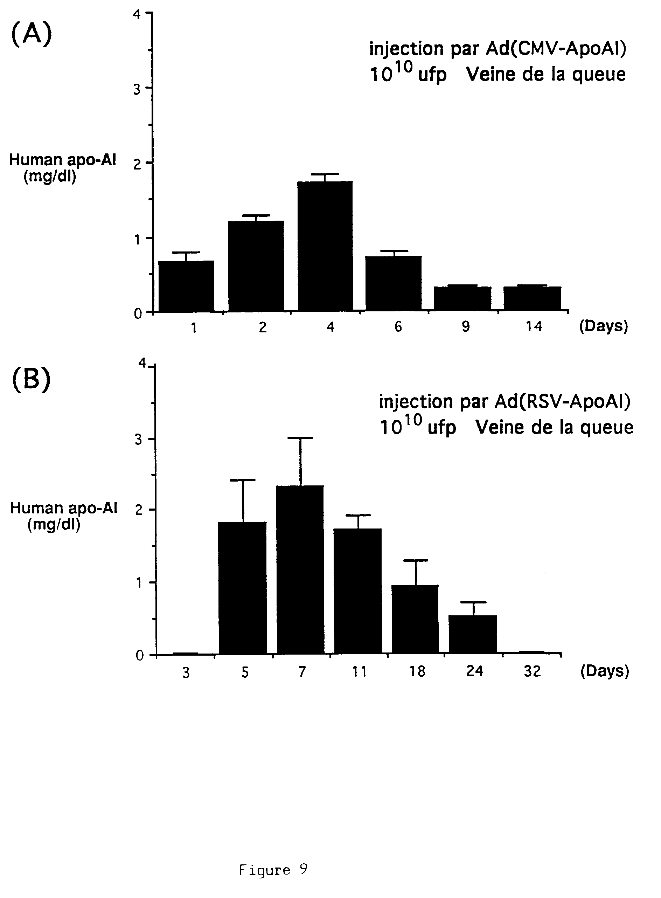

Des niveaux d'ApoAI humaine de l'ordre de 1 à 5 mg/dl sont trouvés dans les plasma de souris injectées par 10¹⁰ ufp des virus Ad (RSV-ApoAI) ou Ad (CMV-ApoAI). Des niveaux équivalents sont trouvés après une injection par la veine de la queue ou par le sinus rétro-orbitaire. Cette expression est stable pendant environ 15 jours dans le cas du promoteur CMV mais dure de l'ordre de 4 semaine dans le cas du promoteur RSV (figure 9).

### Exemple C : Construction d'un adénovirus recombinant défectif contenant un gène antisens de l'apolipoprotéine AII:

### C1. Préparation des plasmides portant un gène antisens de l'apoAII :

### 1. Construction du plasmide pXL2264

Le plasmide pXL2264 contient le cDNA codant pour l'apolipoprotéine A-II humaine.

Il a été construit de la manière suivante :

Le fragment d'ADN correspondant au cDNA de l'apoA-II a été isolé par la technique de RT-PCR à partir des ARN totaux de cellules HepG2. Les cDNA ont été produits par transcription inverse des ARN polyA+ à l'aide d'amorces oligodT. Une PCR a alors été réalisée sur ces cDNA avec les oligonucléotides Sq3319 et Sq3320 (SEQ ID n° 8 et 9), spécifiques de la séquence de l'apoA-II (Chan et coll., Meth. Enzymol. 128 (1986) 745). Le fragment de 473 pb obtenu a été cloné dans le plasmide pCRII (Invitrogen) et sa séquence vérifiée. La partie 5' du brin codant du cDNA est à côté du site HindIII de pCRII et la partie 3' à côté du site XhoI. Le plasmide résultant a été appelé pXL2264.

### 2. Construction du plasmide pXL2267

Le plasmide pXL2267 contient le cDNA codant pour le gène antisens complet de l'apolipoprotéine A-II humaine (SEQ ID n° 12).

C'est un des clones obtenus par clonage du fragment PCR de 473 pb dans le plasmide pCR-II (cf 1 ci-dessus). Sa séquence a été vérifiée. Le cDNA de l'A-II est orienté de manière inversée par rapport au cDNA porté par le plasmide pXL2264 (partie 5' du brin codant à côté du site XhoI et partie 3' proche du site HindIII).

### 3. Construction du plasmide pXL2268

Le plasmide pXL2268 contient le cDNA codant pour un gène antisens partiel de l'apolipoprotéine A-II humaine, dont la séquence est complémentaire à celle du cDNA de l'A-II pour les bases +1 à +95 et +307 à +473 (SEQ ID n° 13).

Il a été construit de la manière suivante :

Deux PCR ont été réalisées sur le plasmide pXL2264 (portant l'A-II sens), l'une avec les oligonucléotides Sq3319 et Sq3369, et l'autre avec les oligonucléotides Sq3320 et Sq3364 (SEQ ID n° 10). Les fragments issus de ces PCR sont respectivement de 96 et 167 pb. Une troisième PCR a été pratiquée sur un mélange de ces deux fragments avec les oligonucléotides Sq3319 et Sq3320. Le fragment de 263 pb obtenu a été cloné dans le plasmide pCRII (Invitrogen) et sa séquence vérifiée. La partie 5' du brin codant du cDNA de l'A-II est à côté du site XhoI de pCRII. Le plasmide résultant a été appelé pXL2268.

### 4. Construction du plasmide pXL2269

Le plasmide pXL2269 est un vecteur d'expression eucaryote contenant le cDNA du gène de l'apolipoprotéine A-II humaine sous le contrôle de la séquence LTR du virus RSV.

Il a été construit de la manière suivante :
- digestion du plasmide pXL2264 par HindIII-XhoI, puis,
- insertion du fragment HindIII-XhoI contenant le cDNA sens de l'apoA-II dans le vecteur pREP4 (Invitrogen) préalablement digéré par les mêmes enzymes.

### 5. Construction des plasmides pXL2270 et pXL2271

Les plasmides pXL2270 et pXL2271 sont des vecteurs d'expression eucaryote contenant les cDNA des gènes antisens respectivement complets et partiels de l'apolipoprotéine A-II humaine, sous le contrôle des régions promotrices du virus CMV.

Ils ont été construits de la manière suivante :
- digestion des plasmides pXL2267 et pXL2268 par HindIII-XhoI, puis,
- insertion des fragments HindIII-XhoI de chaque plasmide, contenant les cDNA antisens de l'apoA-II, dans le vecteur pCEP4 (Invitrogen) préalablement digéré par les mêmes enzymes.

Les plasmides ainsi obtenus ont été désignés respectivement pXL2270 (antisens complet) et pXL2271 (antisens partiel).

### 6. Construction du plasmide pXL2403

Le plasmide pXL2403 est un vecteur d'expression eucaryote contenant notamment l'ADNc codant pour l'apolipoprotéine A-II humaine sous le contrôle du promoteur MLP de l'adénovirus, la séquence signal de polyadénylation et l'origine de réplication du virus SV40.

Il a été construit de la manière suivante :
- digestion du plasmide pXL2264 par ECoRI, puis,
- insertion du fragment ECoRI-ECoRI contenant l'ADNc sens de l'apoA-II dans le vecteur pMT3 (Swick et coll., PNAS 89 (1992) 1812), préalablement linéarisé par ECoRI. Le plasmide, sélectionné pour l'orientation correcte de l'ADNc sens, a été désigné pXL2403.

### 7. Construction du plasmide pXL2404

Le plasmide pXL2404 est un vecteur d'expression eucaryote contenant un ADNc codant pour un ARN messager dont la séquence est complémentaire à la séquence entière de l'ARNm codant pour l'apolipoprotéine A-II humaine. Il a été construit selon la même technique que le plasmide pXL2403 mais il a été sélectionné pour l'orientation en sens opposé de l'ADNc sens. Le plasmide ainsi obtenu a été dénommé pXL2404.

### 8. Construction du plasmide pXL2405

Le plasmide pXL2405 contient l'ADNc codant pour VA RNA_{I}, ARN de l'adénovirus de type 2 dont la transcription est sous la dépendance de l'ARN polymérase III.

Il a été construit de la manière suivante :
- Le fragment d'ADN correspondant au VA RNA_{I} a été isolé par la technique de PCR à partir de l'ADN génomique de l'adénovirus de type 2 au moyen des oligonucléotides VACla5' (SEQ ID n° 14) et VACla3' (SEQ ID n° 15). Le fragment de 238 pb obtenu a été purifié et cloné dans le plasmide pCR-II (Invitrogen). Le plasmide ainsi obtenu a été dénommé pXL2405.

### 9. Construction du plasmide pXL2406

Le plasmide pXL2406 contient l'ADNc codant pour VA RNA_{I}, dont la séquence est modifiée par insertion, en position 102, d'une séquence de 33 nucléotides, complémentaire à la séquence +44/+76 de l'ADNc codant pour l'apoA-II humaine (SEQ ID n° 21).

Il a été construit de la manière suivante :

Deux PCR ont été réalisées sur l'ADN génomique de l'adénovirus de type 2, l'une avec les oligonucléotides Sq3892 (SEQ ID n° 16) et VACla5', et l'autre avec les oligonucléotides VACla3' et Sq3893 (SEQ ID n° 17). Les fragments issus de ces PCR sont respectivement de 162 et 118 pb. Une troisième PCR a été pratiquée sur un mélange de ces deux fragments, après leur purification, au moyen des oligonucléotides VACla5' et VACla3'. Le fragment de 271 pb obtenu a été cloné dans le plasmide pCRII (Invitrogen) et sa séquence vérifiée sur 254 pb (SEQ ID n° 22). Le plasmide résultant a été dénommé pXL2406.

### 10. Construction du plasmide pXL2407

Le plasmide pXL2407 contient l'ADNc codant pour VA RNA_{I}, dont la séquence est modifiée par insertion, en position 102, d'une séquence de 33 nucléotides, complémentaire à la séquence +441/+473 de l'ADNc codant pour l'apoA-II humaine (SEQ ID n° 23).

Il a été construit de la manière suivante :

Deux PCR ont été réalisées sur l'ADN génomique de l'adénovirus de type 2, l'une avec les oligonucléotides VACla5' et Sq3894 (SEQ ID n° 18), et l'autre avec les oligonucléotides Sq3895 (SEQ ID n° 19) et VACla3'. Les fragments issus de ces PCR sont respectivement de 162 et 118 pb. Une troisième PCR a été pratiquée sur un mélange de ces deux fragments, après leur purification, au moyen des oligonucléotides VACla5' et VACla3'. Le fragment de 271 pb obtenu a été cloné dans le plasmide pCRII (Invitrogen) et sa séquence vérifiée sur 254 pb (SEQ ID n° 24). Le plasmide résultant a été dénommé pXL2407.

### 11. Construction des plasmides pXL2408 et pXL2409

Les plasmides pXL2408 et pXL2409 sont des vecteurs d'expression eucaryote contenant un VA RNA_{I} modifié par l'insertion d'une séquence de 33 nucléotides, respectivement complémentaire aux séquences +44/+76 et +441/+473 de l'ADNc codant pour l'apoA-II humaine. De plus, ils contiennent les séquences codant pour les résistances à la néomycine et à la kanamycine.

Ils ont été construits de la manière suivante :
- digestion des plasmides pXL2405 et pXL2406 par ClaI, puis,
- insertion des fragments ClaI-ClaI de chaque plasmide, contenant les VA RNA_{I} modifiés dans le vecteur pVV2, préalablement linéarisé par ClaI. Les plasmides ont été désignés respectivement pXL2408 et pXL2409.

### C2. Fonctionnalité des ARN antisens

Les vecteurs exprimant un ARN antisens (pXL2270, pXL2271, pXL2404, pXL2408, pXL2409) ont été testés:
- pour leur expression, après transfection transitoire dans les cellules COS-1,
- pour leur fonctionnalité par expression transitoire dans les mêmes cellules, après cotransfection avec pXL2403,
- pour leur fonctionnalité par génération de lignées cellulaires 293 transfectées de façon stable.

### 1. Expression des ARN antisens :

- Les vecteurs exprimant les ARN antisens ont été introduits dans les cellules COS-1 par électroporation. 48 heures après transfection, les ARNs totaux ont été extraits des cellules. La présence d'ARNs messager antisens a été détectée par la technique de Northern-blot. Pour cela, les ARNs totaux ont été déposés sur gel d'agarose en présence de formaldéhyde puis transférés sur membrane de nylon. La membrane a été hybridée avec une sonde radiomarquée au phosphore 32. Pour l'expression des VA RNA_{I} modifiés, cette sonde était soit l'ADNc codant pour VA RNA_{I}, soit l'oligonucléotide Sq4112 (SEQ ID n° 20). Pour l'expression de l'ARN antisens complet, cette sonde était soit l'ADNc codant pour l'apoA-II humaine, soit l'oligonucléotide Sq3320 (SEQ ID n° 9). La fixation spécifique de la sonde sur les ARNs de la membrane a été révélée après autoradiographie à -80°C.
Les résultats obtenus montrent que, pour toutes les constructions testées, l'expression des transcrits antisens peut être détectée.

### 2. Expression de l'apolipoprotéine A-II

Le plasmide pXL2403 a été testé pour sa fonctionnalité, par expression dans les cellules COS-1. Pour cela, le plasmide a été introduit dans les cellules par électroporation.
Deux jours après la transfection, les ARNs totaux ont été extraits des cellules et la présence d'ARN messager codant pour l'apoA-II a été détectée par la technique de Northern-blot, à l'aide de sondes spécifiques : soit l'ADNc codant pour l'apoA-II humaine, soit l'oligonucléotide Sq3319 (SEQ ID n° 8).
D'autre part, les surnageants cellulaires ont été récupérés et testés pour leur contenu en apoA-II par un test ELISA. Pour le test ELISA, un anticorps monoclonal dirigé centre l'apoA-II (10 µg/ml en tampon carbonate de pH=9,6) a été fixé sur les puits d'une plaque Immulon II (Dynatech), par incubation d'une nuit à +4°C. Les puits ont alors été saturés une heure à 37°C par de la sérumalbumine bovine (diluée à 2% dans du tampon PBS de pH=7,4). Les surnageants cellulaires purs ou dilués (dilution en PBS-BSA 2%) ont été déposés dans les puits et incubés une heure à 37°C. La révélation a ensuite été effectuée par (i) l'incubation des puits, pendant une heure à 37°C, avec un mélange d'anticorps monoclonaux anti-apoA-II fixés covalemment à la peroxydase, (ii) l'incubation dans l'obscurité en présence du réactif TMB et (iii) lecture en spectrophotométrie à une longueur d'onde de 630nm.
Les résultats obtenus montrent que l'expression de l'apolipoprotéine A-II peut être détectée.

### 3. Génération de lignées cellulaires 293 exprimant de façon stable les ARNs antisens

Les vecteurs exprimant les ARNs antisens ont été introduits dans les cellules 293 par la technique de transfection en présence de phosphate de calcium. Un vecteur portant le gène de résistance à la néomycine a été introduit simultanément avec le vecteur pXL2404. La sélection des clones ayant reçu les vecteurs antisens a été réalisée par culture des cellules en présence de G418 (400 µg/ml). Les clones ont alors été caractérisés et sélectionnés pour la présence de l'ADNc codant pour l'ARN antisens (PCR) et du transcrit antisens (Northern-blot).
Les lignées stables sélectionnées ainsi que la lignée 293 parentale ont ensuite été transfectées par le plasmide pXL2403. 48 heures après la transfection, les surnageants cellulaires ont été récupérés et testés pour leur contenu en apoA-II par un test ELISA (cf 2. ci-dessus).

### C3. Préparation des adénovirus recombinants

Les plasmides préparés en C1 sont ensuite linéarisés et cotransfectés pour la recombinaison avec un vecteur adénoviral déficient, dans les cellules helper (lignée 293) apportant en *trans* les fonctions codées par les régions E1 (E1A et E11B) d'adénovirus, selon le protocole décrit dans l'exemple A3.

### Exemple D : Construction d'un adénovirus recombinant défectif contenant le gène de l'apolipoprotéine E:

### D1. Construction du plasmide pXL2335

Ce plasmide contient l'ADNc de l'isoforme 3 de l'ApoE sous le contrôle du promoteur RSV.

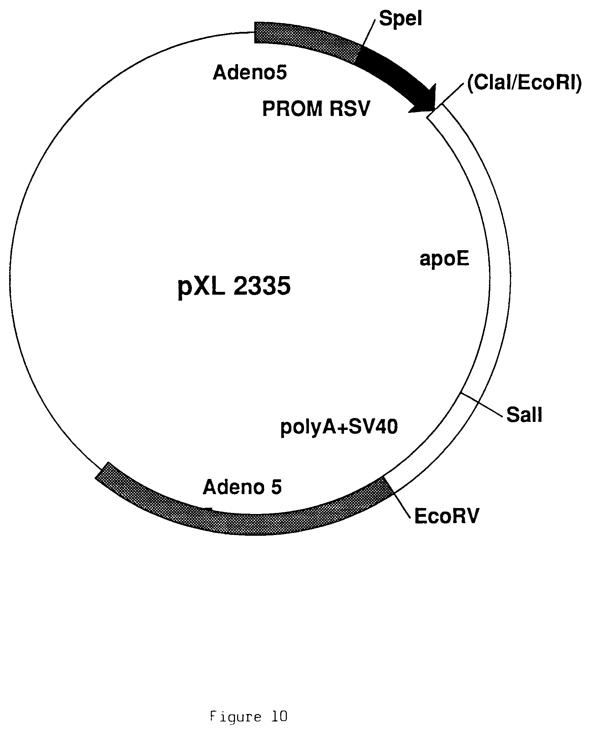

Il a été construit par clonage de l'ADNc contenu au site SmaI d'un plasmide pGEM3, sorti par une coupure EcoRI comblée à la T4 polymérase et une seconde coupure Sal I et inséré dans le plasmide pXL2244 ouvert par ClaI, rempli à la Klenow et recoupé par Sal I (figure 10).

### D2. Préparation des adénovirus recombinants

Le plasmide pXL2335 préparé en D1 est ensuite linéarisé et cotransfecté pour la recombinaison avec un vecteur adénoviral déficient, dans les cellules helper (lignée 293) apportant en *trans* les fonctions codées par les régions E1 (E1A et E11B) d'adénovirus, selon le protocole décrit dans l'exemple A3.

## Revendications

1. Adénovirus recombinant défectif contenant au moins un gène inséré capable de réguler les niveaux d'apolipoprotéine in vivo.

2. Adénovirus selon la revendication 1 caractérisé en ce qu'il est dépourvu des régions de son génome qui sont nécessaires à sa réplication dans la cellule infectée.

3. Adénovirus selon la revendication 2 caractérisée en ce qu'il sagit d'un adénovirus de type Ad 5.

4. Adénovirus selon l'une des revendications 1 à 3 caractérisé en ce qu'il contient au moins un gène inséré codant pour une apolipoprotéine ou pour un produit protéique à activité de type apolipoprotéine.

5. Adénovirus selon la revendication 4 caractérisé en ce que le gène inséré code pour tout ou partie de l'apolipoprotéine AI, de l'apolipoprotéine AIV, de l'apolipoprotéine E ou d'un dérivé ou variant naturel de celles-ci.

6. Adénovirus selon la revendication 5 caractérisé en ce que le gène inséré code pour l'apolipoprotéine AI ou un variant de celle-ci, de préférence, possédant une cystéine en position 173.

7. Adénovirus selon l'une des revendications 1 à 3 caractérisé en ce qu'il contient au moins un gène antisens dont l'expression dans la cellule cible permet de contrôler l'expression de gènes ou la transcription d'ARNm cellulaires codant pour des apolipoprotéines.

8. Adénovirus selon la revendication 7 caractérisée en ce que le gène antisens permet de diminuer les niveaux d'expression d'apolipoprotéine AII.

9. Adénovirus selon l'une des revendications 1 à 8 caractérisée en ce que le gène inséré comprend des séquences permettant son expression dans la cellule infectée.

10. Adénovirus selon l'une des revendications 1 à 9 caractérisée en ce que le gène inséré comprend une séquence signal dirigeant le polypeptide synthétisé dans les voies de sécrétion de la cellule cible.

11. Utilisation d'un adénovirus selon l'une des revendications 1 à 10 pour la préparation d'une composition pharmaceutique destinée au traitement ou à la prévention des pathologies liées aux dyslipoprotéinémies.

12. Utilisation selon la revendication 11 pour la préparation d'une composition pharmaceutique destinée au traitement de l'athérosclérose et/ou des maladies neurologiques..

13. Composition pharmaceutique comprenant un ou plusieurs adénovirus recombinants défectifs selon l'une des revendications 1 à 10.

14. Composition pharmaceutique selon la revendication 13 caractérisée en ce qu'elle se présente sous forme injectable.

15. Composition pharmaceutique selon la revendication 13 caractérisée en ce qu'elle comprend entre 10⁴ et 10¹⁴ pfu/ml, et de préférence 10⁶ à 10¹⁰ pfu/ml d'adénovirus recombinant défectif.

## Claims

1. Defective recombinant adenovirus containing at least one inserted gene capable of regulating the apolipoprotein levels in vivo.

2. Adenovirus according to claim 1, characterized in that it lacks the regions of its genome which are necessary for its replication in the infected cell.

3. Adenovirus according to claim 2, characterized in that it is a type Ad 5 adenovirus.

4. Adenovirus according to one of claims 1 to 3, characterized in that it contains at least one inserted gene which encodes an apolipoprotein or a protein product with apolipoprotein type activity.

5. Adenovirus according to claim 4, characterized in that the inserted gene encodes all or part of the apolipoprotein AI, the apolipoprotein AIV, the apolipoprotein E or a derivative or natural variant thereof.

6. Adenovirus according to claim 5, characterized in that the inserted gene encodes the apolipoprotein AI or a variant thereof, preferably having a cystein in position 173.

7. Adenovirus according to one of claims 1 to 3, characterized in that it contains at least one antisense gene whose expression in the target cell makes it possible to control the expression of genes or the transcription of cellular mRNA encoding apolipoproteins.

8. Adenovirus according to claim 7, characterized in that the antisense gene makes it possible to reduce the levels of expression of apolipoprotein AII.

9. Adenovirus according to one of claims 1 to 8, characterized in that the inserted gene comprises sequences permitting its expression in the infected cell.

10. Adenovirus according to one of claims 1 to 9, characterized in that the inserted gene comprises a signal sequence directing the synthesized polypeptide in the secretion pathways of the target cell.

11. Use of an adenovirus according to one of claims 1 to 10, for the preparation of a pharmaceutical composition intended for the treatment or the prevention of pathologies linked to dyslipoproteinemias.

12. Use according to claim 11, for the preparation of a pharmaceutical composition intended for the treatment of atherosclerosis and/or neurological diseases.

13. Pharmaceutical composition comprising one or more defective recombinant adenoviruses according to one of claims 1 to 10.

14. Pharmaceutical composition according to claim 13, characterized in that it is in injectable form.

15. Pharmaceutical composition according to claim 13, characterized in that it comprises between 10⁴ and 10¹⁴ pfu/ml, and preferably 10⁶ to 10¹⁰ pfu/ml of defective recombinant adenovirus.

## Patentansprüche

1. Defektives rekombinantes Adenovirus, das mindestens ein insertiertes Gen enthält, das die Apolipoproteingehalte in vivo regulieren kann.

2. Adenovirus nach Anspruch 1, dadurch **gekennzeichnet**, daß ihm die Regionen seines Genoms fehlen, die für seine Replikation in der infizierten Zelle notwendig sind.

3. Adenovirus nach Anspruch 2, dadurch **gekennzeichnet**, daß es ein Adenovirus vom Typ Ad5 ist.

4. Adenovirus nach einem der Ansprüche 1 bis 3, dadurch **gekennzeichnet**, daß es mindestens ein insertiertes Gen enthält, das ein Apolipoprotein oder ein Proteinprodukt mit Aktivität vom Apolipoproteintyp codiert.

5. Adenovirus nach Anspruch 4, dadurch **gekennzeichnet**, daß das insertierte Gen die Gesamtheit oder einen Teil von Apolipoprotein AI, Apolipoprotein AIV, Apolipoprotein E oder eines Derivats oder natürlicher Variante davon codiert.

6. Adenovirus nach Anspruch 5, dadurch **gekennzeichnet**, daß das insertierte Gen Apolipoprotein AI oder eine Variante davon, bevorzugt eine, die ein Cystein in Position 173 besitzt, codiert.

7. Adenovirus nach einem der Ansprüche 1 bis 3, dadurch **gekennzeichnet**, daß es mindestens ein Antisensgen enthält, dessen Expression in der Zielzelle die Kontrolle der Expression von Genen oder die Transkription zellulärer mRNA, die Apolipoproteine codieren, erlaubt.

8. Adenovirus nach Anspruch 7, dadurch **gekennzeichnet**, daß das Antisensgen die Verringerung der Expressionshöhen von Apolipoprotein A II erlaubt.

9. Adenovirus nach einem der Ansprüche 1 bis 8, dadurch **gekennzeichnet**, daß das insertierte Gen Sequenzen umfaßt, die dessen Expression in der infizierten Zelle erlauben.

10. Adenovirus nach einem der Ansprüche 1 bis 9, dadurch **gekennzeichnet**, daß das insertierte Gen eine Signalsequenz umfaßt, die das synthetisierte Polypeptid durch die Sekretionswege der Zielzelle steuert.

11. Verwendung eines Adenovirus nach einem der Ansprüche 1 bis 10 zur Herstellung einer pharmazeutischen Zusammensetzung zur Behandlung oder zur Verhütung von Krankheitsbildern im Zusammenhang mit Dyslipoproteinämien.

12. Verwendung nach Anspruch 11 zur Herstellung einer pharmazeutischen Zusammensetzung zur Behandlung von Atherosklerose und/oder neurologischen Erkrankungen.

13. Pharmazeutische Zusammensetzung, umfassend ein oder mehrere defektive rekombinante Adenoviren nach einem der Ansprüche 1 bis 10.

14. Pharmazeutische Zusammensetzung nach Anspruch 13, dadurch **gekennzeichnet**, daß sie in injizierbarer Form vorliegt.

15. Pharmazeutische Zusammensetzung nach Anspruch 13, dadurch **gekennzeichnet**, daß sie 10⁴ bis 10¹⁴ KBE/ml und bevorzugt 10⁶ bis 10¹⁰ KBE/ml defektives rekombinantes Adenovirus enthält.


LISTE DE SEQUENCES

(1) INFORMATION GENERALE:

(i) DEPOSANT:
(A) NOM: RHONE-POULENC RORER S.A.
(B) RUE: 20, avenue Raymond ARON
(C) VILLE: ANTONY
(E) PAYS: FRANCE
(F) CODE POSTAL: 92165

(ii) TITRE DE L' INVENTION: : VIRUS RECOMBINANTS ET LEUR
UTILISATION EN THERAPIE GENIQUE.

(iii) NOMBRE DE SEQUENCES: 24

(iv) FORME LISIBLE PAR ORDINATEUR:
(A) TYPE DE SUPPORT: Tape
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(2) INFORMATION POUR LA SEQ ID NO: 1:

(i) CARACTERISTIQUES DE LA SEQUENCE:
(A) LONGUEUR: 61 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq1851

(xi) DESCRIPTION DE LA SEQUENCE: SEQ ID NO: 1:

TCTGGCAGCA AGATGAAACC CCCCAGAGCC CCTGGGATCG AGTGAAGGAC CTGGCCACTG T 61

(2) INFORMATION POUR LA SEQ ID NO: 2:

(i) CARACTERISTIQUES DE LA SEQUENCE:
(A) LONGUEUR: 33 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq1852

(xi) DESCRIPTION DE LA SEQUENCE: SEQ ID NO: 2:

CGCGGATCCG CGCAGCTTCT CTTGCAGCTC GTG 33

(2) INFORMATION POUR LA SEQ ID NO: 3:

(i) CARACTERISTIQUES DE LA SEQUENCE:
(A) LONGUEUR: 55 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq1849

(xi) DESCRIPTION DE LA SEQUENCE: SEQ ID NO: 3:

GGGGAAGCTT ATGAAAGCTG CGGTGCTGAC CTTGGCCGTG CTCTTCCTGA CGGGG 55

(2) INFORMATION POUR LA SEQ ID NO: 4:

(i) CARACTERISTIQUES DE LA SEQUENCE:
(A) LONGUEUR: 61 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq1850

(xi) DESCRIPTION DE LA SEQUENCE: SEQ ID NO: 4:

GGGGGGGGTT CATCTTGCTG CCAGAAATGC CGAGCCTGGC TCCCCGTCAG GAAGACGACG G 61

(2) INFORMATION POUR LA SEQ ID NO: 5:

(i) CARACTERISTIQUES DE LA SEQUENCE:
(A) LONGUEUR: 20 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq3561

(xi) DESCRIPTION DE LA SEQUENCE: SEQ ID NO: 5:

TGCTGACCTT GGCCGTGCTC 20

(2) INFORMATION POUR LA SEQ ID NO: 6:

(i) CARACTERISTIQUES DE LA SEQUENCE:
(A) LONGUEUR: 20 paires de bases
(B) TYPE: acide nucléique
(C) NOMBRE DE BRINS: simple

(D) CONFIGURATION: linéaire

(ii) TYPE DE MOLECULE: ADNc

(vi) ORIGINE:
(A) ORGANISME: OLIGONUCLEOTIDE sq3571

(xi) DESCRIPTION DE LA SEQUENCE: SEQ ID NO: 6:

CGCAGCGGCT CCACCTTCTG 20

(2) INFORMATION POUR LA SEQ ID NO: 7:

(i) CARACTERISTIQUES DE LA SEQUENCE:
(A) LONGUEUR: 20 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq3570

(xi) DESCRIPTION DE LA SEQUENCE: SEQ ID NO: 7:

GGACAGAGCT GATCCTTGAA 20

(2) INFORMATION POUR LA SEQ ID NO: 8:

(i) CARACTERISTIQUES DE LA SEQUENCE:
(A) LONGUEUR: 29 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE sq3319

(xi) DESCRIPTION DE LA SEQUENCE: SEQ ID NO: 8:

GGATTCATTC AGCATTTATT GTAGCAAAG 29

(2) INFORMATION POUR LA SEQ ID NO: 9:

(i) CARACTERISTIQUES DE LA SEQUENCE:
(A) LONGUEUR: 30 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
      (A) ORGANISME: OLIGONUCLEOTIDE sq3320

(xi) DESCRIPTION DE LA SEQUENCE: SEQ ID NO: 9:

AGGCACAGAC ACCAAGGACA GAGACGCTGG                                    30

(2) INFORMATION POUR LA SEQ ID NO: 10:

(i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire

(ii) TYPE DE MOLECULE: ADNc

(vi) ORIGINE:
      (A) ORGANISME: OLIGONUCLEOTIDE sq3364

(xi) DESCRIPTION DE LA SEQUENCE: SEQ ID NO: 10:

CTCAAGAAGT TAACCTGGTG AGGAGTAGCA                                    30

(2) INFORMATION POUR LA SEQ ID NO: 11:

(i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire

(ii) TYPE DE MOLECULE: ADNc

(vi) ORIGINE:
      (A) ORGANISME: OLIGONUCLEOTIDE sq3369

(xi) DESCRIPTION DE LA SEQUENCE: SEQ ID NO: 11:

TGCTACTCCT CACCAGGTTA ACTTCTTGAG                                    30

(2) INFORMATION POUR LA SEQ ID NO: 12:

(i) CARACTERISTIQUES DE LA SEQUENCE:
      (A) LONGUEUR: 473 paires de bases
      (B) TYPE: acide nucléique
      (C) NOMBRE DE BRINS: simple
      (D) CONFIGURATION: linéaire

(ii) TYPE DE MOLECULE: ADNc

(iv) ANTISENS : Antisens complet anti-apoAII

(xi) DESCRIPTION DE LA SEQUENCE: SEQ ID NO: 12:

GGATTCATTC AGCATTTATT GTAGCAAAGA GTGGGTAGGG ACAGGAGCTC TAGGACTGGC 60

CAGTGGGTGT TCTAGAGGCC AGCTGGGGTT GGAAGACAAT GGTCTGGACA CTTCACTGGG 120

TGGCAGGCTG TGTTCCAAGT TCCACGAAAT AGCTCAAGAA GTTAACCAGT TCCGTTCCAG 180

CCTTCTTGAT CAGGGGTGTC AGCTGCTCCT TTGACTTTTC AAAGTAAGAC TTGGCCTCGG 240

CCTGAAGCTC TGGGCTCTTG ACCTTCTCCA TCAGGTCCTT GCCATAGTCA GTCACGGTCT 300

GGAAGTACTG AGAAACCAGG CTCTCCACAC ATGGCTCCTT TGCCTGTCTC CGAACCAAAG 360

CTCCTTCAAG GCTGCAGATG GTGAGGAGTA GCACAGTTGC TGCGAGCAGC TTCATGTTGG 420

TAACAGTGGG GAGGGCGGCC TAGCCAGCGT CTCTGTCCTT GGTGTCTGTG CCT 473

(2) INFORMATION POUR LA SEQ ID NO: 13:

(i) CARACTERISTIQUES DE LA SEQUENCE:
(A) LONGUEUR: 262 paires de bases
(B) TYPE: acide nucléique
(C) NOMBRE DE BRINS: simple
(D) CONFIGURATION: linéaire

(ii) TYPE DE MOLECULE: ADNc

(iv) ANTISENS : Antisens partiel anti-apoAII

(xi) DESCRIPTION DE LA SEQUENCE: SEQ ID NO: 13:

GGATTCATTC AGCATTTATT GTAGCAAAGA GTGGGTAGGG ACAGGAGCTC TAGGACTGGC 60

CAGTGGGTGT TCTAGAGGCC AGCTGGGGTT GGAAGACAAT GGTCTGGACA CTTCACTGGG 120

TGGCAGGCTG TGTTCCAAGT TCCACGAAAT AGCTCAAGAA GTTAACCTGG TGAGGAGTAG 180

CACAGTTGCT GCGAGCAGCT TCATGTTGGT AACAGTGGGG AGGGCGGCCT AGCCAGCGTC 240

TCTGTCCTTG GTGTCTGTGC CT 262

(2) INFORMATION POUR LA SEQ ID NO: 14:

(i) CARACTERISTIQUES DE LA SEQUENCE:
(A) LONGUEUR: 32 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE VACla5'

(xi) DESCRIPTION DE LA SEQUENCE: SEQ ID NO: 14:

GTTATCGATC TAGACCGTGC AAAAGGAGAG CC 32

(2) INFORMATION POUR LA SEQ ID NO: 15:

(i) CARACTERISTIQUES DE LA SEQUENCE:
(A) LONGUEUR: 33 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:
(A) ORGANISME: OLIGONUCLEOTIDE VACla3'

(xi) DESCRIPTION DE LA SEQUENCE: SEQ ID NO: 15:

CTTCCAGGCG CGGCGGCTGC TGCGATCGAT TTT 33

(2) INFORMATION POUR LA SEQ ID NO: 16:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

(A) ORGANISME: OLIGONUCLEOTIDE Sq3892

(xi) DESCRIPTION DE LA SEQUENCE: SEQ ID NO: 16:

GCCGTGATCC ATGCGGTTTG CTGCGAGCAG CTTCATGTT 39

(2) INFORMATION POUR LA SEQ ID NO: 17:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

(A) ORGANISME: OLIGONUCLEOTIDE Sq3893

(xi) DESCRIPTION DE LA SEQUENCE: SEQ ID NO: 17:

CTTCATGTTG GTAACAGTGG GACCGCCCGC GTGTCGAAC 39

(2) INFORMATION POUR LA SEQ ID NO: 18:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

(A) ORGANISME: OLIGONUCLEOTIDE Sq3894

(xi) DESCRIPTION DE LA SEQUENCE: SEQ ID NO: 18:

GCCGTGATCC ATGCGGTTGG ATTCATTCAG CATTTATTG 39

(2) INFORMATION POUR LA SEQ ID NO: 19:

(i) CARACTERISTIQUES DE LA SEQUENCE:
(A) LONGUEUR: 39 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

(A) ORGANISME: OLIGONUCLEOTIDE Sq3895

(xi) DESCRIPTION DE LA SEQUENCE: SEQ ID NO: 19:

CATTTATTGT AGCAAAGAGT GACCGCCCGC GTGTCGAAC 39

(2) INFORMATION POUR LA SEQ ID NO: 20:

(i) CARACTERISTIQUES DE LA SEQUENCE:
(A) LONGUEUR: 30 paires de bases
(B) TYPE: acide nucléique
(C) NOMBRE DE BRINS: simple
(D) CONFIGURATION: linéaire

(ii) TYPE DE MOLECULE: ADNc

(vi) ORIGINE:

(A) ORGANISME: OLIGONUCLEOTIDE Sq4112

(xi) DESCRIPTION DE LA SEQUENCE: SEQ ID NO: 20:

AAAAGGAGCG CTCCCCCGTT GTCTGACGTC 30

(2) INFORMATION POUR LA SEQ ID NO: 21:

(i) CARACTERISTIQUES DE LA SEQUENCE:
(A) LONGUEUR: 33 paires de bases
(B) TYPE: acide nucléique
(C) NOMBRE DE BRINS: simple
(D) CONFIGURATION: linéaire

(ii) TYPE DE MOLECULE: ADNc

(iv) ANTISENS : Antisens partiel anti-apoAII, complémentaire de
la séquence +44/+76.

(xi) DESCRIPTION DE LA SEQUENCE: SEQ ID NO: 21:

TGCTGCGAGC AGCTTCATGT TGGTAACAGT GGG 33

(2) INFORMATION POUR LA SEQ ID NO: 22:

(i) CARACTERISTIQUES DE LA SEQUENCE:
(A) LONGUEUR: 254 paires de bases
(B) TYPE: acide nucléique
(C) NOMBRE DE BRINS: simple
(D) CONFIGURATION: linéaire

(ii) TYPE DE MOLECULE: ADNc

(iv) ANTISENS : Antisens partiel anti-apoAII, cloné dans pXL2406.

(xi) DESCRIPTION DE LA SEQUENCE: SEQ ID NO: 22:

TCTAGACCGT GCAAAAGGAG AGCCTGTAAG CGGGCACTCT TCCGTGGTCT GGTGGATAAA 60

TTCGCAAGGG TATCATGGCG GACGACCGGG GTTCGAACCC CGGATCCGGC CGTCCGCCGT 120

GATCCATGCG GTTTGCTGCG AGCAGCTTCA TGTTGGTAAC AGTGGGACCG CCCGCGTGTC 180

GAACCCAGGT GTGCGACGTC AGACAACGGG GGAGCGCTCC TTTTGGCTTC CTTCCAGGCG 240

CGGCGGCTGC TGCG 254

(2) INFORMATION POUR LA SEQ ID NO: 23:

(i) CARACTERISTIQUES DE LA SEQUENCE:
(A) LONGUEUR: 33 paires de bases
(B) TYPE: acide nucléique
(C) NOMBRE DE BRINS: simple
(D) CONFIGURATION: linéaire

(ii) TYPE DE MOLECULE: ADNc

(iv) ANTISENS : Antisens partiel anti-apoAII, complémentaire de
la séquence +441/+473.

(xi) DESCRIPTION DE LA SEQUENCE: SEQ ID NO: 23:

GGATTCATTC AGCATTTATT GTAGCAAAGA GTG 33

(2) INFORMATION POUR LA SEQ ID NO: 24:

(i) CARACTERISTIQUES DE LA SEQUENCE:
(A) LONGUEUR: 254 paires de bases
(B) TYPE: acide nucléique
(C) NOMBRE DE BRINS: simple
(D) CONFIGURATION: linéaire

(ii) TYPE DE MOLECULE: ADNc

(iv) ANTISENS : Antisens partiel anti-apoAII, cloné dans pXL2407.

(xi) DESCRIPTION DE LA SEQUENCE: SEQ ID NO: 24:

TCTAGACCGT GCAAAAGGAG AGCCTGTAAG CGGGCACTCT TCCGTGGTCT GGTGGATAAA 60

TTCGCAAGGG TATCATGGCG GACGACCGGG GTTCGAACCC CGGATCCGGC CGTCCGCCGT 120

GATCCATGCG GTTGGATTCA TTCAGCATTT ATTGTAGCAA AGAGTGACCG CCCGCGTGTC 180

GAACCCAGGT GTGCGACGTC AGACAACGGG GGAGCGCTCC TTTTGGCTTC CTTCCAGGCG 240

CGGCGGCTGC TGCG 254